(19) Europäisches Patentamt
European Patent Office
Office européen des brevets

(11) **EP 3 903 760 A1**

(12) **EUROPEAN PATENT APPLICATION**
published in accordance with Art. 153(4) EPC

(43) Date of publication:
**03.11.2021 Bulletin 2021/44**

(51) Int Cl.:
***A61K 6/831*** (2020.01)     ***A61K 6/887*** (2020.01)

(21) Application number: **19902830.9**

(22) Date of filing: **27.12.2019**

(86) International application number:
**PCT/JP2019/051551**

(87) International publication number:
**WO 2020/138472 (02.07.2020 Gazette 2020/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2018  JP 2018246073**

(71) Applicant: **Kuraray Noritake Dental Inc.**
**Kurashiki-shi, Okayama 710-0801 (JP)**

(72) Inventor: **HIRAMATSU, Shogo**
**Tainai-shi, Niigata 959-2653 (JP)**

(74) Representative: **D Young & Co LLP**
**120 Holborn**
**London EC1N 2DY (GB)**

(54) **DENTAL MILL BLANK HAVING OPTIMIZED COLOR TONE**

(57) The present invention provides a dental mill blank that can be fabricated, through a milling process, into a highly aesthetic dental prosthesis having an opalescent quality characteristic of natural teeth and also having a sufficient chroma level. The present invention relates to a dental mill blank comprising: 40 mass% or more of an inorganic filler, wherein the dental mill blank satisfies the following conditions (1) and (2):
(1) a specimen with a thickness of 1.20 $\pm$ 0.01 mm has a chromaticity satisfying $-0.5 \leq a^*_{black} \leq 2.5$ and $12.0 \leq b^*_{black} \leq 25.0$ in the L*a*b* color system when measured against a black background; and
(2) the specimen with a thickness of 1.20 $\pm$ 0.01 mm has an opalescence value of 17.5 or more.

EP 3 903 760 A1

**Description**

TECHNICAL FIELD

The present invention relates to a dental mill blank for use in fabrication of a dental crown using a milling machine based on three-dimensional coordinate data.

BACKGROUND ART

In recent years, more and more dental prostheses such as inlays and crowns are fabricated using a CAD/CAM system, which designs the dental prostheses using a computer and fabricates them through a milling process using a milling device. In fabrication using such a system, a block having an appropriate size and a shape of a cuboid, cylinder, or disc is provided. The block is set in a milling machine and milled to yield a restoration in a shape of a dental crown or a dental bridge. As the material of the block, various materials have been proposed, including glass ceramics, zirconia, titanium, acrylic resins, and composite materials containing a polymer resin(s) and an inorganic filler(s).

Restoration treatments of defects in crowns and dentition are required to provide the appearance as close as possible to the color tone of natural tissue. Restorations fabricated by milling blocks formed of a single component often fail to meet such an aesthetic requirement. On this account, as a block to be subjected to a milling process, blocks including multiple layers with different color tones have been proposed (Patent Literatures 1 to 3, for example).

Patent Literature 1 discloses a resin-based block including a dentin restorative resin layer and an enamel restorative resin layer. By defining the contrast ratio and the light diffusing performance of the dentin restorative resin layer, the block can achieve reproduction of a color tone similar to that of a natural tooth while the block has a simple two-layer structure.

Patent Literature 2 discloses, as a resin block for CAD/CAM, a dental mill blank in the form of a laminate including three or more layers. In the dental mill blank, the chromaticity difference $\Delta E^*$ and the transparency difference $\Delta\Delta L^*$ between the uppermost layer and the lowermost layer, and also, the chromaticity difference $\Delta E^*$ and the transparency difference $\Delta\Delta L^*$ between each pair of adjacent layers are defined. This allows the dental mill blank to be fabricated into a dental prosthesis having a color tone and transparency that are more similar to those of a natural tooth.

Patent Literature 3 discloses a dental mill blank in the form of a laminate including two or more layers. The respective layers in the laminate satisfy specific correlations between the contrast and the color tone, whereby a resin block for dental milling superior in terms of aesthetics can be obtained.

CITATION LIST

Patent Literatures

    Patent Literature 1: JP 2014-161440 A
    Patent Literature 2: WO 2018/074605 A1
    Patent Literature 3: JP 2017-105764 A

SUMMARY OF INVENTION

Technical Problem

The above-described conventional techniques are all directed to the color tone of a mill blank material used in dental CAD/CAM technology. These conventional techniques, however, still have room for improvement in terms of color tone design, and there remains a problem in that, when a fabricated prosthesis is placed in the oral cavity of a patient, the chroma level thereof drops in the dark oral environment, resulting in mismatch with adjacent teeth. In addition, it was found that the opalescent quality of natural teeth cannot be reproduced by these conventional techniques. Accordingly, there is a problem in that, in order to impart the opalescent quality characteristic of natural teeth to the dental prosthesis, a dental technician has to perform an additional operation such as applying a porcelain material or the like onto the prosthesis.

In light of the foregoing, an object of the present invention is to provide a dental mill blank that can be fabricated, through a milling process, into a highly aesthetic dental prosthesis having an opalescent quality characteristic of natural teeth and also having a sufficient chroma level.

Solution to Problem

The present inventor conducted intensive studies to find a solution to the foregoing issues, and found that the above-described problems can be solved with a dental mill blank that satisfies specific conditions concerning the chromaticity measured against a black background and has a specific opalescence value.
Specifically, the present invention includes the following.

[1] A dental mill blank comprising:

40 mass% or more of an inorganic filler,
wherein the dental mill blank satisfies the following conditions (1) and (2):

(1) a specimen with a thickness of $1.20 \pm 0.01$ mm has a chromaticity satisfying $-0.5 \leq a^*_{black} \leq 2.5$ and $12.0 \leq b^*_{black} \leq 25.0$ in the L*a*b* color system when measured against a black background; and
(2) the specimen with a thickness of $1.20 \pm 0.01$ mm has an opalescence value of 17.5 or more.

[2] The dental mill blank according to [1], wherein the dental mill blank has a layered structure comprising two or more layers, and at least one of the layers satisfies the following conditions (1) and (2):

(1) a specimen with a thickness of $1.20 \pm 0.01$ mm has a chromaticity satisfying $-0.5 \leq a^*_{black} \leq 2.5$ and $12.0 \leq b^*_{black} \leq 25.0$ in the L*a*b* color system when measured against a black background; and
(2) the specimen with a thickness of $1.20 \pm 0.01$ mm has an opalescence value of 17.5 or more.

[3] The dental mill blank according to [1] or [2], wherein the dental mill blank has a layered structure comprising two or more layers, at least one of the layers satisfies the following conditions (1) and (2), and at least one other layer satisfies the following condition (3):

(1) a specimen with a thickness of $1.20 \pm 0.01$ mm has a chromaticity satisfying $-0.5 \leq a^*_{black} \leq 2.5$ and $12.0 \leq b^*_{black} \leq 25.0$ in the L*a*b* color system when measured against a black background;
(2) the specimen with a thickness of $1.20 \pm 0.01$ mm has an opalescence value of 17.5 or more; and
(3) a specimen with a thickness of $1.20 \pm 0.01$ mm has a chromaticity satisfying $-2.0 \leq a^*_{black} \leq 0.0$ and $3.0 \leq b^*_{black} \leq 16.0$ in the L*a*b* color system when measured against a black background.

[4] The dental mill blank according to any one of [1] to [3], wherein the inorganic filler comprises an inorganic filler having an average particle size of 30 to 700 nm.
[5] The dental mill blank according to any one of [1] to [4], further comprising an inorganic pigment,
wherein the content of the inorganic pigment is 0.070 to 0.100 parts by mass relative to 100 parts by mass of the inorganic filler.
[6] The dental mill blank according to [5], wherein the inorganic pigment comprises three or more types of inorganic pigments.
[7] The dental mill blank according to any one of [1] to [6], wherein the inorganic filler comprises an inorganic filler having an average particle size of 30 to 180 nm.

Advantageous Effects of Invention

A dental mill blank of the present invention can be fabricated, through a milling process, into a highly aesthetic dental prosthesis having an opalescent quality characteristic of natural teeth and also having a sufficient chroma level. According to the present invention, the mill blank can be fabricated into a dental prosthesis that achieves a high level of aesthetics in a dark oral environment even when it has a single-layer structure, and the mill blank can be fabricated into a dental prosthesis with a higher degree of reproducibility of the aesthetic appearance of a natural tooth when it has a layered structure.

DESCRIPTION OF EMBODIMENTS

It is important that a dental mill blank of the present invention contain 40 mass% or more of an inorganic filler and satisfy the following conditions (1) and (2):

(1) a specimen with a thickness of $1.20 \pm 0.01$ mm has a chromaticity satisfying $-0.5 \leq a^*_{black} \leq 2.5$ and $12.0 \leq$

$b^*_{black} \leq 25.0$ in the L*a*b* color system when measured against a black background; and
(2) the specimen with a thickness of $1.20 \pm 0.01$ mm has an opalescence value of 17.5 or more.

Typically, the level of chroma can be increased by increasing the amount of a pigment added to an inorganic filler. However, when the content of the pigment is high, the transparency of a dental prosthesis to be obtained is lowered, resulting in a deteriorated opalescent quality of the dental prosthesis. To address this issue, a dental mill blank of the present invention has suitable features that enable both the improvement in chroma level and the appearance of an opalescent quality. This is described in greater details below.

In a dental mill blank of the present invention, it is important that a specimen with a thickness of $1.20 \pm 0.01$ mm have a chromaticity satisfying $-0.5 \leq a^*_{black} \leq 2.5$ and $12.0 \leq b^*_{black} \leq 25.0$ in the L*a*b* color system when measured against a black background (the condition (1)). When the dental mill blank satisfies the condition (1), a dental prosthesis obtained by milling the dental mill blank can have a chroma level sufficient for visual recognition even in a dark oral environment. The chromaticity preferably satisfies $0.5 \leq a^*_{black} \leq 2.0$ and $12.0 \leq b^*_{black} \leq 20.0$. The chromaticity in the present invention can be measured by the method described in the EXAMPLES section below.

In a dental mill blank of the present invention, it is important that the specimen with a thickness of $1.20 \pm 0.01$ mm have an opalescence value of 17.5 or more (the condition (2)). The opalescence value is defined by the following formula using chromaticity values in the L*a*b* color system, namely, an $a^*_{white}$ value and a $b^*_{white}$ value measured against a white background and an $a^*_{black}$ value and a $b^*_{black}$ value measured against a black background.

$$(\text{Opalescence value}) = \{(a^*_{white} - a^*_{black})^2 + (b^*_{white} - b^*_{black})^2\}^{1/2}$$

It is known that natural teeth have an opalescence value of 22.9. The dental mill blank satisfying the condition (2) can be fabricated, through a milling process, into a highly aesthetic dental prosthesis that has an opalescent quality characteristic of (in particular, the incisal area of) natural teeth. The term "opalescent quality" means exhibiting an opal effect, and the "opal effect" means a unique visible light scattering effect like that of an opal. More specifically, the "opal effect" means, when an object contain particles having a size roughly equivalent to the wavelength of light, the particles scatter visible light in the short-wavelength region, thereby rendering transmitted light from the object yellowish and scattered light from the object bluish. The opalescence value is preferably close to 22.9, which is the opalescence value of natural teeth. More specifically, the opalescence value is preferably 17.9 or more and 25.0 or less. The upper limit is not limited to any particular value, as far as judging from the effect expected to be obtained. The opalescence value in the present invention can be measured by the method described in the EXAMPLES section below.

As described above, it is important that a dental mill blank of the present invention contain 40 mass% or more of an inorganic filler and satisfy the conditions (1) and (2). Such a dental mill blank can be obtained by the following production method, for example. The dental mill blank can be obtained by pressing an inorganic filler containing an inorganic pigment in a mold (dry molding), bringing the thus-obtained molded body into contact with a polymerizable monomer containing a polymerization initiator, and then curing the molded body through polymerization. Alternatively, as another example of the production method, the dental mill blank also can be obtained by preparing a paste-like material containing, as main components, an inorganic filler, a polymerizable monomer, a polymerization initiator, and an inorganic pigment and then curing the paste-like material through polymerization in a mold having a desired shape. The respective components that can be used in a dental mill blank of the present invention are described below.

In view of the strength of a dental mill blank to be obtained, it is important that the content of the inorganic filler used in the present invention be 40 mass% or more relative to the total mass of the dental mill blank. The content is preferably 50 mass% or more, even more preferably 55 mass% or more, and may be 60 mass% or more. Also, in a dental mill blank of the present invention, the content of the inorganic filler relative to the total mass of the dental mill blank is preferably 95 mass% or less, more preferably 90 mass% or less, and may be 85 mass%. The content of the inorganic filler can be measured using a known method, and examples of the method include the method described in the EXAMPLES section below.

The inorganic filler preferably contains a nanofiller having an average particle size of 30 to 700 nm. The smaller the average particle size, the greater the light scattering, which causes a dental prosthesis to be obtained to exhibit a higher degree of opalescent quality. The average particle size is more preferably 30 to 180 nm, even more preferably 30 to 100 nm. By setting the average particle size to fall within the above range, a dental mill blank to be obtained can be adjusted to satisfy the condition (2), and such a dental mill blank can be fabricated into a dental prosthesis having an opalescent quality characteristic of natural teeth. In a dental mill blank of a certain embodiment, the inorganic filler preferably consists of a nanofiller having an average particle size of 30 to 700 nm, more preferably consists of a nanofiller having an average particle size of 30 to 180 nm, and even more preferably consists of a nanofiller having an average particle size of 30 to 100 nm.

In the present specification, the average particle size of an inorganic filler means the average particle size of primary particles (i.e., the average primary particle size) of the filler, and can be determined by a laser diffraction scattering method or by electron microscopy of the particles. Specifically, the laser diffraction scattering method is convenient for measurement of the average particle size of particles of 100 nm or more, and the electron microscopy is convenient for measurement of the average particle size of ultra-fine particles of less than 100 nm. In the above, 100 nm is a value determined by the laser diffraction scattering method. The measurement by the laser diffraction scattering method can be performed using, for example, a laser diffraction particle size analyzer (SALD-2300, manufactured by Shimadzu Corporation) with a 0.2% sodium hexametaphosphate aqueous solution used as dispersion medium. The measurement by electron microscopy can be performed by, for example, taking a micrograph of particles with a scanning electron microscope (S-4000, manufactured by Hitachi, Ltd.) and measuring the particle size of particles (200 or more) observed in a unit field of view of the micrograph with an image-analyzing particle size distribution analysis software (Mac-View, manufactured by Mountech Co., Ltd.). In this case, the particle size of each particle is obtained as an arithmetic mean value of the longest and shortest dimensions thereof, and the average primary particle size is calculated from the number of the particles and their particle sizes thus obtained.

The type of the inorganic filler is not limited as long as the present invention can exhibit its effects, and known inorganic fillers can be used. Specific examples of the inorganic filler include conventionally known inorganic fillers made of various types of glass (containing, as a main component, silicon dioxide (quartz, quartz glass, silica gel, or the like) or silicon and further containing boron and/or aluminum together with any of various types of heavy metal), alumina, various types of ceramics, diatomaceous earth, kaolin, clay minerals (such as montmorillonite), activated white clay, synthetic zeolites, mica, silica, calcium fluoride, ytterbium fluoride, calcium phosphate, barium sulfate, zirconium dioxide (zirconia), titanium oxide, and hydroxyapatite. Examples of the inorganic filler may include organic-inorganic composite particles (organic-inorganic composite filler) obtained by previously adding a polymerizable monomer to the above-described inorganic particles to obtain a mixture in a paste form, curing the paste through polymerization, and then pulverizing the cured paste into particles. As the inorganic filler, any one type of the above-described inorganic particles or organic-inorganic composite particles may be used alone, or two or more types of them may be used in combination.

The inorganic filler may be previously surface-treated. Surface-treating inorganic particles with a surface treatment agent improves the molding density during press molding to improve the strength of a mill blank, and also improves the compatibility with a polymerizable monomer to allow the composition of the inorganic filler and the polymerizable monomer to easily turn into a paste. The surface treatment agent may be a known surface treatment agent, examples of which include: organometallic compounds such as organosilicon compounds, organotitanium compounds, organozirconium compounds, and organoaluminum compounds; and acidic group-containing compounds having at least one acidic group such as a phosphoric acid group, a pyrophosphoric acid group, a thiophosphoric acid group, a phosphonic acid group, a sulfonic acid group, or a carboxylic acid group. When two or more types of surface treatment agents are used, a mixture of these two or more types of surface treatment agents may be used to form a surface treatment layer, or a plurality of surface treatment layers may be layered to form a multilayer structure. As the surface treatment method, known methods can be used without any particular limitation.

Specific examples of the organosilicon compounds include methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyl tris($\beta$-methoxyethoxy)silane, 3,3,3-trifluoropropyl trimethoxysilane, methyl-3,3,3-trifluoropropyl dimethoxysilane, $\beta$-(3,4-epoxycyclohexyl)ethyltrimethoxysilane, $\gamma$-glycidoxypropyltrimethoxysilane, $\gamma$-glycidoxypropylmethyldiethoxysilane, $\gamma$-glycidoxypropyltriethoxysilane, $\gamma$-methacryloxypropylmethyldimethoxysilane, $\gamma$-methacryloxypropylmethyldiethoxysilane, N-($\beta$-aminoethyl)-$\gamma$-aminopropylmethyldimethoxysilane, N-($\beta$-aminoethyl)-$\gamma$-aminopropyltrimethoxysilane, N-($\beta$-aminoethyl)-$\gamma$-aminopropyltriethoxysilane, $\gamma$-aminopropyltrimethoxysilane, $\gamma$-aminopropyltriethoxysilane, N-phenyl-$\gamma$-aminopropyltrimethoxysilane, $\gamma$-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, $\omega$-(meth)acryloxyalkyl trimethoxysilane (having 3 to 12 carbon atoms between a (meth)acryloxy group and a silicon atom, such as, for example, $\gamma$-methacryloxypropyltrimethoxysilane), and $\omega$-(meth)acryloxyalkyl triethoxysilane (having 3 to 12 carbon atoms between a (meth)acryloxy group and a silicon atom such as, for example, $\gamma$-methacryloxypropyltriethoxysilane. In the present invention, "(meth)acryloxy" is used as a term that encompasses both "methacryloxy" and "acryloxy".

The inorganic filler in the present invention preferably contains an inorganic pigment. The inorganic pigment is not limited as long as the present invention can exhibit its effects, and known pigments used in dental prostheses can be used without any limitation. Examples of such an inorganic pigment include: chromates such as chrome yellow, zinc yellow, and barium yellow; ferrocyanides such as iron blue; sulfides such as vermilion, cadmium yellow, zinc sulfide, antimony white, and cadmium red; sulfates such as barium sulfate, zinc sulfate, and strontium sulfate; oxides such as zinc white, titanium oxide, iron oxide red (red oxide), iron oxide black, iron oxide yellow, and chromium oxide; hydroxides such as aluminum hydroxide; silicates such as calcium silicate and ultramarine; and carbons such as carbon black and graphite. One type of such pigments may be used alone, or two or more types of them may be used in combination.

The pigment may be selected as appropriate according to the desired color tone of a dental mill blank. Of these pigments, titanium oxide, red oxide, iron oxide black, iron oxide yellow, etc., which are inorganic pigments having, e.g., excellent heat resistance or light resistance, are particularly preferred for a dental mill blank of the present invention. A dental mill blank of a certain preferred embodiment is one that contains 40 mass% or more of an inorganic filler, further contains three or more types of inorganic pigments, and satisfies the conditions (1) and (2). A dental mill blank of another preferred embodiment is one that contains 40 mass% or more of an inorganic filler, further contains four types of inorganic pigments, and satisfies the conditions (1) and (2).

The inorganic pigment content is preferably 0.028 to 0.070 mass%, more preferably 0.028 to 0.060 mass%, relative to the total mass of the dental mill blank. The inorganic pigment content is preferably 0.070 to 0.100 parts by mass, more preferably 0.070 to 0.085 parts by mass, relative to 100 parts by mass (total amount) of the inorganic filler. By setting the content to fall within the above range, a dental mill blank to be obtained can be adjusted to satisfy the condition (1), and such a dental mill blank can be fabricated into a dental prosthesis having a sufficient chroma level. When the dental mill blank has, for example, a layered structure including two or more layers, it is only required that the inorganic pigment content in at least one layer should fall within the above range.

When the inorganic filler contains the inorganic pigment, the inorganic filler and the particulate inorganic pigment may be mixed together to achieve uniform dispersion. As a method for mixing the inorganic filler and the inorganic pigment to achieve uniform dispersion, known methods for mixing and dispersing powder may be used without any limitation, and the method may be either a dry method or a wet method. In order to mix the inorganic filler and the inorganic pigment in such a manner that their particles are more uniformly dispersed, it is preferable to disperse the inorganic filler and the inorganic pigment in the presence of a solvent and then remove or distill the solvent. Methods known in the art can be used to cause dispersion. For example, a disperser such as a sand mill, a bead mill, an attritor, a colloid mill, a ball mill, an ultrasonic crusher, a homomixer, a dissolver, or a homogenizer can be used. Dispersion conditions vary depending on, for example, the particle size and the amount of the inorganic filler or the inorganic pigment to be used, the type and the added amount of the solvent, or the type of the disperser. According to the dispersing state of particles of the inorganic filler or the inorganic pigment, the dispersion conditions such as the dispersing time, a stirrer, and the number of revolutions can be selected as appropriate. The solvent used for wet dispersion is preferably water and/or a solvent compatible with water. Examples of the solvent include alcohols (e.g., ethanol, methanol, and isopropanol), ethers, and ketones (e.g., acetone and methyl ethyl ketone). As a method for distilling the solvent, either distillation under reduced pressure using a rotary evaporator or drying using a spray dryer may be selected. In view of mass production, drying using a spray dryer is preferable.

The polymerizable monomer is described below. As the polymerizable monomer in the present invention, known polymerizable monomers used for, for example, dental composite resins can be used without any limitation. Typically, radical polymerizable monomers are suitably used. Specific examples of the radical polymerizable monomers include esters of α-cyanoacrylic acid, (meth)acrylic acid, α-halogenated acrylic acid, crotonic acid, cinnamic acid, sorbic acid, maleic acid, itaconic acid, and the like, (meth)acrylamide, (meth)acrylamide derivatives, vinyl esters, vinyl ethers, mono-N-vinyl derivatives, and styrene derivatives. Of these, (meth)acrylic acid esters and (meth)acrylamide derivatives are preferable, and (meth)acrylic acid esters are more preferable. In the present invention, "(meth)acryl" is used as a term that encompasses both "methacryl" and "acryl". Examples of (meth)acrylate-based polymerizable monomers and (meth)acrylamide derivative-based polymerizable monomers are given below.

(I) Monofunctional (meth)acrylates and (meth)acrylamide derivatives

Examples of monofunctional (meth)acrylates and (meth)acrylamide derivatives include methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, N-methylol (meth)acrylamide, N-hydroxyethyl (meth)acrylamide, N,N-bis(2-hydroxyethyl) (meth)acrylamide, (meth)acryloyloxide decylpyridinium bromide, (meth)acryloyloxide decylpyridinium chloride, (meth)acryloyloxyhexadecylpyridinium chloride, (meth)acryloyloxydecylammonium chloride, and 10-mercaptodecyl (meth)acrylate.

(II) Bifunctional (meth)acrylates

Examples of bifunctional (meth)acrylates include: ethylene glycol di(meth)acrylate, triethylene glycol di(meth)acrylate, propylene glycol di(meth)acrylate, neopentyl glycol di(meth)acylate, 1,6-hexanediol di(meth)acrylate, 1,10-decanediol di(meth)acrylate, 2,2-bis[4-(3-acryloyloxy-2-hydroxypropoxy)phenyl]propane, 2,2-bis[4-(3-methacryloyloxy-2-hydroxypropoxy)phenyl]propane (commonly known as "Bis-GMA"), 2,2-bis[4-(meth)acryloyloxyethoxyphenyl]propane, 2,2-bis[4-(meth)acryloyloxypolyethoxyphenyl]propane, 1,2-bis[3-(meth)acryloyloxy-2-hydroxypropoxy]ethane,pentaerythritol di(meth)acrylate, [2,2,4-trimethylhexamethylene bis(2-carbamoyloxyethyl)]dimethacrylate (commonly known as "UDMA"), and 2,2,3,3,4,4-hexafluoro-1,5-pentyl di(meth)acrylate.

(III) Trifunctional or higher-functional (meth)acrylates

Examples of trifunctional or higher-functional (meth)acrylates include trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarboxy)propane-1,3-diol] tetra(meth)acrylate, and 1,7-diacryloyloxy-2,2,6,6-tetraacryloyloxymethyl-4-oxaheptane.

In addition to these (meth)acrylic acid ester-based and (meth)acrylamide derivative-based polymerizable monomers, oxirane compounds and oxetane compounds that can be cationically polymerized also can be suitably used. One type of such polymerizable monomers may be used alone, or two or more types of them may be used in combination. The polymerizable monomer used in the present invention is preferably in a liquid state, but need not necessarily be in a liquid state at ordinary temperatures. Further, a solid polymerizable monomer also can be used in a state where it is mixed with and dissolved in another polymerizable monomer that is in a liquid state. The viscosity range of the polymerizable monomer (measured at 25°C) is preferably 10 Pa·s or less, more preferably 5 Pa·s or less, even more preferably 2 Pa·s or less. In the case where two or more types of polymerizable monomers are mixed together to obtain a dissolved state or such a mixture is further diluted with a solvent, it is not necessary that the viscosity of each type of polymerizable monomer fall within the above range, and the viscosity of the composition obtained by mixing and dissolving them preferably falls within the above range.

Next, a polymerization initiator used to cure the polymerizable monomer by causing polymerization to obtain a polymer is described. The polymerization initiator can be selected from polymerization initiators for general industrial applications. In particular, polymerization initiators used for dental applications are preferably used. Examples of such polymerization initiators include thermal polymerization initiators, photopolymerization initiators, and chemical polymerization initiators. One type of polymerization initiator may be used alone, or two or more types of polymerization initiators may be used in any suitable combination.

Examples of the thermal polymerization initiators include organic peroxides and azo compounds. Examples of the organic peroxides include ketone peroxides, hydroperoxides, diacyl peroxides, dialkyl peroxides, peroxyketals, peroxyesters, and peroxydicarbonates. Examples of the ketone peroxides include methyl ethyl ketone peroxide, methyl isobutyl ketone peroxide, methylcyclohexanone peroxide, and cyclohexanone peroxide. Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne. Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

Examples of the peroxyesters include α-cumyl peroxyneodecanoate, t-butyl peroxyneodecanoate, t-butyl peroxypivarate, 2,2,4-trimethylpentylperoxy-2-ethyl hexanoate, t-amylperoxy-2-ethylhexanoate, t-butylperoxy-2-ethylhexanoate, di-t-butylperoxy isophthalate, di-t-butylperoxy hexahydroterephthalate, t-butylperoxy-3,3,5-trimethyl hexanoate, t-butylperoxy acetate, t-butylperoxy benzoate, and t-butylperoxymaleic acid.

Examples of the peroxydicarbonates include di-3-methoxy peroxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

Of these organic peroxides, diacyl peroxides are preferably used in view of an overall balance among the safety, storage stability, and radical formation potential, and in particular, benzoyl peroxide is more preferably used.

Examples of the azo compounds include 2,2'-azobis(isobutyronitrile), 2,2'-azobis(2,4-dimethylvaleronitrile), 4,4'-azobis(4-cyanovaleric acid), 1,1'-azobis(cyclohexane-1-carbonitrile), dimethyl-2,2'-azobis(isobutyrate), and 2,2'-azobis(2-aminopropane)dihydrochloride.

Examples of the photopolymerization initiators include (bis)acylphosphine oxides, α-diketones, and coumarins.

Concerning the above-described (bis)acylphosphine oxides, examples of acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, and their salts. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and their salts. Of these (bis)acylphosphine oxides, 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt are preferable.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Of these, camphorquinone is suitable.

Examples of the coumarins include compounds described in JP 09(1997)-003109 A and JP 10(1998)-245525 A, such as 3,3'-carbonyl bis(7-diethylaminocoumarin), 3-(4-methoxybenzoyl)coumarin, 3-thienoyl coumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 7-methoxy-3-(p-nitrobenzoyl)coumarin, 3-(p-nitrobenzoyl)coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazole-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetram ethyl-1H,5H,11H-[1]benzopyrano[6,7,8-ij]quinolizine-11-one, and 10-(2-benzothiazolyl)-2,3,6,7-tetrahydro-1,1,7,7-tetramethyl-1H,5H,11H-[1]benzopyr ano[6,7,8-ij]quinolizine-11-one.

Of the above-describedcoumarins, 3,3'-carbonyl bis(7-diethylaminocoumarin) and 3,3'-carbonylbis(7-dibutylaminocoumarin) are suitable.

Of these photopolymerization initiators, it is preferable to use at least one selected from the group consisting of (bis)acylphosphine oxides, α-diketones, and coumarins, which are widely used for dental curable compositions.

By using such a photopolymerization initiator optionally in combination with a polymerization accelerator, photopolymerization may be efficiently performed in a shorter time.

Major examples of a polymerization accelerator suitably used in combination with a photopolymerization initiator include tertiary amines, aldehydes, compounds having a thiol group(s), and sulfinic acids and their salts.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, 2-(methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethylmethacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of the aldehydes include dimethylaminobenzaldehyde and terephthalaldehyde. Examples of the compounds having a thiol group(s) include 2-mercaptobenzoxazole, decanethiol, 3-mercaptopropyltrimethoxysilane, and thiobenzoic acid.

Examples of the sulfinic acid and their salts include benzenesulfinic acid, sodium benzenesulfinate, potassium benzenesulfinate, calcium benzenesulfinate, lithium benzenesulfinate, p-toluenesulfinic acid, sodium p-toluenesulfinate, potassium p-toluenesulfinate, calcium p-toluenesulfinate, lithium p-toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, and calcium 2,4,6-triisopropylbenzenesulfinate.

Examples of preferably used chemical polymerization initiators include: organic peroxides and amines; and redox polymerization initiators such as organic peroxides, and sulfinic acids (or their salts). When a redox polymerization initiator is used, an oxidizing agent and a reducing agent thereof, which should be packaged separately, need to be mixed together immediately before use. The oxidizing agent of the redox polymerization initiator may be an organic peroxide, for example. The organic peroxide used as the oxidizing agent of the redox polymerization initiator is not limited to any particular one, and known organic peroxides can be used. Specific examples of the organic peroxide include those given above as examples of the thermal polymerization initiator.

Of these organic peroxides, diacyl peroxides are preferably used in view of an overall balance among the safety, storage stability, and radical formation potential, and in particular, benzoyl peroxide is more preferably used.

As the reducing agent of the redox polymerization initiator, a tertiary aromatic amine having no electron-withdrawing group on its aromatic ring is typically used. Examples of the tertiary aromatic amine having no electron-withdrawing group on its aromatic ring include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, and N,N-bis(2-hydroxyethyl)-3,5-di-t-butylaniline.

Such a chemical polymerization initiator may be used optionally in combination with a polymerization accelerator. The polymerization accelerator for the chemical polymerization initiator can be selected from polymerization accelerators for general industrial applications. In particular, polymerization accelerators used for dental applications are preferably used. One type of polymerization accelerator may be used alone, or two or more types of polymerization accelerators may be used in any suitable combination. Specific examples of the polymerization accelerator include amines, sulfinic acids and their salts, copper compounds, and tin compounds.

The amines used as the polymerization accelerator for the chemical polymerization initiator are classified into aliphatic amines and aromatic amines having an electron-withdrawing group on their aromatic rings. Examples of the aliphatic amines include: primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. Of these, tertiary aliphatic amines are preferable in view of curability and storage stability of resulting compositions, and in particular, N-methyldiethanolamine and triethanolamine are used more preferably.

Examples of the tertiary aromatic amine having an electron-withdrawing group on their aromatic rings and used as the polymerization accelerator for the chemical polymerization initiator include ethyl 4-(N,N-dimethylamino)benzoate, methyl 4-(N,N-dimethylamino)benzoate, n-butoxyethyl 4-(N,N- dimethylamino)benzoate, 2-(methacryloyloxy)ethyl 4-N,N-dimethylaminobenzoate, 4-(N,N-dimethylamino)benzophenone, and butyl 4-(N,N-dimethylamino)benzoate. Of these, at least one selected from the group consisting of N,N-di(2-hydroxyethyl)-p-toluidine, ethyl 4-(N,N-dimethylamino)benzoate, n-butoxyethyl 4-(N,N-dimethylaminobenzoate), and 4-(N,N-dimethylamino)benzophenone is preferably used in view of capability of imparting excellent curability to resulting compositions.

Examples of the sulfinic acids and their salts used as the polymerization accelerator include those given above as examples of the polymerization accelerator for the photopolymerization initiator, and sodium benzenesulfinate, sodium p-toluenesulfinate, and sodium 2,4,6-triisopropylbenzenesulfinate are preferable.

Examples of the copper compounds that are suitably used as the polymerization accelerator include copper acetylacetonate, cupric acetate, copper oleate, cupric chloride, and cupric bromide.

Examples of the tin compounds used as the polymerization accelerator include di-n-butyltin dimalate, di-n-octyltin dimalate, di-n-octyltin dilaurate, and di-n-butyltin dilaurate. Particularly preferable tin compounds are di-n-octyltin dilaurate and di-n-butyltin dilaurate.

Of the above-described photopolymerization initiators, it is preferable to use a photopolymerization initiator and a thermal polymerization initiator in combination, and the combination of a (bis)acyl phosphine oxide and diacyl peroxide is more preferable.

The content of the polymerization initiator is not limited to any particular value. In view of, for example, the curability of the resulting composition, the content of the polymerization initiator is preferably 0.001 to 30 parts by mass relative to 100 parts by mass (total amount) of the polymerizable monomer. When the content of the polymerization initiator is 0.001 parts by mass or more relative to 100 parts by mass of the polymerizable monomer, polymerization proceeds sufficiently to prevent the risk of deteriorated mechanical strength. The content of the polymerization initiator is more suitably 0.05 parts by mass or more, even more suitably 0.1 parts by mass or more. On the other hand, when the content of the polymerization initiator is 30 parts by mass or less relative to 100 parts by mass of the polymerizable monomer, sufficient mechanical strength can be obtained even if the polymerization performance of the polymerization initiator itself is low, and also, the occurrence of precipitation from the composition is prevented. The content is more suitably 20 parts by mass or less.

A dental mill blank of the present invention is preferably processed into an appropriate size settable in a commercially available dental CAD/CAM system. Examples of a preferable size include, but not limited to: a 40 mm × 20 mm × 15 mm prism, which is suitable for fabrication of a bridge for replacement of one missing tooth; a 17 mm × 10 mm × 10 mm prism, which is suitable for fabrication of an inlay or an onlay; a 14 mm × 18 mm × 20 mm prism, a 10 mm × 12 mm × 15 mm prism, and a 14.5 mm × 14.5 mm × 18 mm prism, which are suitable for fabrication of a full crown; and disc having a diameter of 100 mm and a thickness of 10 to 28 mm, which is suitable for fabrication of a long-span bridge or a denture base.

A dental mill blank of the present invention may have a single-layer structure or may have a layered structure including two or more layers. In view of achieving a higher degree of aesthetics in a dark oral environment, the dental mill blank preferably has a layered structure. When a dental mill blank of the present invention has a layered structure including two or more layers, it is preferable that at least one of the layers satisfy the above conditions (1) and (2). This allows the dental mill blank to be fabricated, through a milling process, into a highly aesthetic dental prosthesis having an opalescent quality characteristic of natural teeth and also having a sufficient chroma level.

When the dental mill blank has a layered structure including two or more layers, it is preferable that at least one of the layers satisfy the conditions (1) and (2) and at least one other layer satisfy the following condition (3):

(3) a specimen with a thickness of $1.20 \pm 0.01$ mm has a chromaticity satisfying $-2.0 \leq a^*_{black} \leq 0.0$ and $3.0 \leq b^*_{black} \leq 16.0$ in the L*a*b* color system when measured against a black background.

By using the layer(s) satisfying the conditions (1) and (2) in combination with the at least one other layer satisfying the condition (3), a dental prosthesis (in particular, the incisal area thereof) obtained by milling the dental mill blank can have an aesthetic appearance closer to those of natural teeth. Thus, a highly aesthetic dental prosthesis having an opalescent quality characteristic of natural teeth and also having a sufficient chroma level can be obtained through a milling process. The chromaticity of the at least one other layer preferably satisfies $-1.7 \leq a^*_{black} \leq -0.8$ and $5.0 \leq b^*_{black} \leq 13.0$. The dental mill blank having a layered structure including two or more layers, in which at least one of the layer satisfies the conditions (1) and (2) and at least one other layer satisfies the condition (3) is preferably fabricated into a prosthesis for an anterior tooth (central incisor, lateral incisor, or canine), because the incisal area thereof can have an aesthetic appearance closer to those of natural teeth.

Preferred conditions concerning the respective components (such as the type and the content of the inorganic pigment and the type, the content, and the average particle size of the inorganic filler) to allow at least one of the layers to satisfy the conditions (1) and (2) are the same as described above. On the other hand, in order to allow at least one other layer to satisfy the condition (3), it is preferable that, for example, the content of the inorganic pigment in the at least one other layer be 0.050 to 0.070 to 100 parts by mass, more preferably 0.057 to 0.064 parts by mass, relative to 100 parts by mass (total amount) of the inorganic filler.

In a dental mill blank of a certain preferred embodiment, in view of achieving a higher degree of color tone reproducibility, the $a^*_{black}$ and $b^*_{black}$ values in the condition (1) of the layer(s) satisfying the conditions (1) and (2) are set to be greater than the $a^*_{black}$ and $b^*_{black}$ values of the other layer(s) satisfying the condition (3).

When a dental mill blank of the present invention has a layered structure including two or more layers, the method for producing the dental mill blank may be, for example, a method in which inorganic fillers with different chromaticities are layered and then compressed by pressing to obtain a molded body, which is brought into contact with a polymerizable monomer and then cured (press-impregnation method) or a method in which pastes with different chromaticities, each prepared by mixing an inorganic filler and a polymerizable monomer, are layered and then cured.

A dental mill blank of the present invention may contain any other component(s) as long as the effects of the present invention are not impaired. For example, the dental mill blank may contain an organic fluorescent agent such as diethyl 2,5-dihydroxyterephthalate and a stabilizer such as 2-(2'-hydroxy-3'-t-butyl-5'-methylphenyl)-5-chlorobenzotriazole. The content of the other component(s) in the total mass of the dental mill blank is preferably 0.006 mass% or less, more preferably 0.003 mass% or less.

By milling a dental mill blank of the present invention, a highly aesthetic dental prosthesis having an opalescent quality characteristic of natural teeth and also having a sufficient chroma level can be obtained. Examples of the dental prosthesis include crown restorations such as inlays, onlays, veneers, crowns, and bridges, and also include abutment teeth, dental posts, dentures, denture bases, and implant members (fixtures and abutments). The milling process is preferably performed using, for example, a commercially available dental CAD/CAM system, and examples of such a CAD/CAM system include a CEREC system manufactured by Sirona Dental Systems Inc. and "Katana® system" manufactured by Kuraray Noritake Dental Inc.

EXAMPLES

The following describes the present invention more specifically by way of Examples. It should be noted that the present invention is in no way limited by the following Examples, and various changes may be made by a person with ordinary skill in the art within the technical idea of the present invention.

Measurement of Content (Mass%) of Inorganic Filler

The content of an inorganic filler can be determined by heating an obtained mill blank at high temperature in an electric furnace or the like and then measuring the change in mass before and after removal of organic components (i.e., the ignition residue). Specifically, in the present example, a platinum crucible was precisely weighed (m 1), about 1 g of a sample was added to the platinum crucible, and then, the platinum crucible was precisely weighed (m2). Thereafter, the platinum crucible was ignited in an electric furnace at 575°C ± 25°C for 60 ± 3 minutes. The platinum crucible was then taken out from the electric furnace, left to cool down in a desiccator for 30 minutes, and precisely weighed (m3). Then, the amount of the ignition residue was determined as per the following formula.

$$\text{Ignition residue (\%)} = (m3 - m1)/(m2 - m1) \times 100$$

Measurement of Chromaticity and Opalescence Value

From each of dental mill blanks in a prism shape of 14.5 mm × 18.0 mm × 14.5 mm obtained in Examples and Comparative Examples to described below, a 1.6 mm thick chromaticity plate of 14.5 mm × 18.0 mm was cut out using a diamond cutter (ISOMET-1000). The chromaticity plate was finished with waterproof abrasive paper (#1000, #2000, #3000) to a thickness of 1.20 ± 0.01 mm to obtain a specimen (n = 1). The L*a*b* values (JIS Z 8781-4:2013, Colorimetry-Part 4: CIE 1976 L*a*b* color space) of the polished plate-shaped specimen were measured against a white background using a spectrophotometer (CM-3610A: Konica Minolta, Inc., D65 illuminant, geometric condition c (di: 8°, de: 8°), diffused illumination: 8-degree viewing, measurement mode: SCI, measurement area $\Phi$:illumination area $\Phi$= 8 mm : 11 mm). The L*a*b* values of the specimen were measured in the same manner against a black background. Thereafter, the difference $\Delta a^*$ between the chroma values measured against the white background and the black background, the opalescence value, etc. were calculated in the following manner. Measurement against a white background means measurement performed with a standard white plate (L* = 98.68, a* = -0.07, b* = -0.31) being placed behind a specimen to make the background of the specimen white (i.e., to make the side opposite to a measuring instrument with respect to the specimen white), and measurement against a black background means measurement performed with a standard black plate (L* = 25.64, a*= -0.19, b* = -0.80) being placed behind a specimen to make the background of the specimen black (i.e., to make the side opposite to a measuring instrument with respect to the specimen black). Concerning a dental mill blank having a layered structure including two or more layers, chromaticity plates were obtained from the respective layers.

$$\Delta L^* = L^*_{black} - L^*_{white}$$

$$\Delta a^* = a^*_{black} - a^*_{white}$$

$$\Delta b^* = b^*_{black} - b^*_{white}$$

$$(\text{Opalescence value}) = \{(a^*_{white} - a^*_{black})^2 + (b^*_{white} - b^*_{black})^2\}^{1/2}$$

In the above formula, $L^*_{white}$, $a^*_{white}$, and $b^*_{white}$ indicate the results of the measurement against the white background, and $L^*_{black}$, $a^*_{black}$, and $b^*_{black}$ indicate the results of the measurement against the black background.

Evaluation of Color Tone Reproducibility, Opalescent Quality, and Aesthetics of Dental Prosthesis)

Each of the dental mill blanks obtained in Examples and Comparative Examples to described below was milled using a dental CAD/CAM milling machine "DWX-51D" (manufactured by Roland DG Corporation), whereby the dental mill blank was fabricated into a crown for the maxillary right first anterior tooth (n = 1). The crown thus fabricated was trial-fitted in the oral cavity. The color tone reproducibility and opalescent quality of the crown as a dental prosthesis were evaluated through visual observation by five human evaluators, and as comprehensive evaluation considering the color tone reproducibility and the opalescent quality, the aesthetics of the crown was evaluated according to the following evaluation criteria.

The color tone reproducibility of the trial-fitted dental prosthesis was evaluated using a VITA shade guide. The color tone reproducibility of the dental prosthesis was determined as "Excellent: very high color tone reproducibility" when the number of evaluators who evaluated the color tone reproducibility as good was five out of five, determined as "Good: high color tone reproducibility" when the number was four, determined as "Acceptable: an acceptable level of color tone reproducibility" when the number was three, and determined as "Poor: defective color tone reproducibility" when the number was two or less.

The opalescent quality was determined as "Good: favorable opalescent quality" when the number of evaluators who felt the trial-fitted dental prosthesis as bluish was four or more out of five, and determined as "Poor: absence of opalescent quality" when the number was three or less.

The aesthetics was determined as "Excellent: very high degree of aesthetics" when the color tone reproducibility was Excellent and the opalescent quality was Good, determined as "Good: high degree of aesthetics" when both the color tone reproducibility and the opalescent quality were Good or when the color tone reproducibility was Acceptable and the opalescent quality was Good, and determined as "Poor: there is room for improvement in terms of aesthetics" in the other cases.

Production Example of Inorganic Filler

30 g of commercially available ultra-fine silica (manufactured by Nippon Aerosil Co., Ltd., Aerosil® OX 50, average primary particle size: 40 nm, refractive index: 1.46, BET specific surface area: 50 $m^2$/g) was dispersed in 120 mL of water to prepare a dispersion. A solution that had been prepared previously by stirring a mixture of 1.5 g of $\gamma$-methacryloxypropyltrimethoxysilane, 15 mL of water, and 0.108 g of acetic acid was added to this dispersion, and the obtained mixture was stirred at room temperature for 1 hour. Subsequently, inorganic pigments (Japanese Pharmacopoeia titanium oxide, iron oxide black, iron oxide red (red oxide), and iron oxide yellow) were added thereto in the amounts indicated in Table 1, and the obtained mixture was stirred at room temperature for 10 minutes. The solvent was distilled off from the solution under reduced pressure, and the residue was further dried at 90°C for 3 hours. Through the above-described surface treatment, surface-treated inorganic fillers A to G, J, K, N, O, and P were obtained. The added amount of each inorganic pigment was adjusted according to a desired shade. Further, as inorganic fillers for forming intermediate layers when preparing a laminate, an inorganic filler H was prepared by dry-mixing the inorganic fillers G and J at a mass ratio of 2 : 1, and an inorganic filler I was prepared by dry-mixing the inorganic fillers G and J at a mass ratio of 1 : 2. Likewise, an inorganic filler L was prepared by dry-mixing the inorganic fillers K and N at a mass ratio of 2 : 1, and an inorganic filler M was prepared by dry-mixing the inorganic fillers K and N at a mass ratio of 1 : 2.

Further, 30 g of commercially available barium boroaluminosilicate glass powder (manufactured by Schott AG, GM27884, (RegisteredTrademark) UF 2.0, average particle size: 2.0 pm, crushed form) was dispersed in 120 mL of water. The surface treatment was performed in the same manner as described above, whereby inorganic fillers Q, R, and T were obtained. Further, an inorganic filler S was prepared by dry-mixing the inorganic fillers R and T at a mass ratio of 1 : 1.

Preparation Example of Polymerizable Monomer-Containing Composition

A polymerizable monomer-containing composition (m) was prepared by dissolving 1.5 part by mass of benzoyl peroxide, which also serves as a thermal polymerization initiator, in a mixture of 70 parts by mass of [2,2,4-trimethylhexamethylene bis(2-carbamoyloxyethyl)]dimethacrylate (UDMA) and 30 parts by mass of triethylene glycol dimethacrylate (TEGDMA).

Example 1

4.5 g of the inorganic filler A was spread over a lower punch rod of a rectangular press mold of 14.5 × 18.0 mm. The inorganic filler A was subjected to tapping for even spreading. Thereafter, an upper punch rod was set at a predetermined position, and uniaxial pressing (pressing pressure: 60 MPa (16 kN), time period: 1 minute) was performed using a press machine. The upper and lower punch rods were removed from the mold, and a molded body having a single-layer structure was taken out. Thereafter, the molded body was placed inside a polyethylene bag, and the polymerizable monomer-containing composition (m) was added to the bag. Then, the pressure in the bag was reduced to impregnate the molded body with the polymerizable monomer-containing composition (m). The bag was allowed to stand still for one day at room temperature under reduced pressure. Then, using a hot air dryer, the bag was heated at 55°C for 18 hours and further heated at 110°C for 3 hours to polymerize the polymerizable monomer. As a result, a dental mill blank having a desired single-layer structure was obtained. The obtained dental mill blank had a prism shape of 14.5 mm × 18.0 mm × 14.5 mm, and the amount of the ignition residue was 60 mass%.

Examples 2 and 3

Dental mill blanks of Examples 2 and 3 having a single-layer structure were obtained in the same manner as in Example 1, except that, instead of the inorganic filler A, the inorganic fillers B and C were used, respectively. The obtained dental mill blanks both had a prism shape of 14.5 mm × 18.0 mm × 14.5 mm, and the amount of the ignition residue in each of the dental mill blanks was 60 mass%.

Example 4

6.2 g of the inorganic filler D was added little by little to 3.8 g of the polymerizable monomer-containing composition (m) while kneading to prepare a paste-like material. The paste-like material was added to a Teflon® container of 14.5 mm × 14.5 mm × 18 mm until the thickness of the paste-like material reached 15 mm on a 14.5 mm × 18 mm surface. Thereafter, the paste-like material was thermally polymerized at 50°C for 24 hours and subsequently at 110°C for 12 hours in the container. The polymerized material was taken out of the contain after the completion of the polymerizing step. As a result, a dental mill blank having a desired single-layer structure was obtained. The obtained dental mill blank had a prism shape of 14.5 mm × 18.0 mm × 14.5 mm, and the amount of the ignition residue was 61 mass%.

Example 5

Paste-like materials 1 and 2 were prepared by adding 6.2 g of the inorganic filler D and 6.2 g of the inorganic filler F, respectively, little by little to 3.8 g of the polymerizable monomer-containing composition (m) while kneading. The paste-like material 1 was added to a Teflon® container of 14.5 mm × 14.5 mm × 18 mm until the thickness of the paste-like material 1 reached 7.5 mm on a 14.5 mm × 18 mm surface. Then, the paste-like material 2 was further added thereon until the thickness of the paste-like material 2 reached 7.5 mm. As a result, a pre-polymerized laminate contained in the container was obtained. Thereafter, the laminate was thermally polymerized at 50°C for 24 hours and subsequently at 110°C for 12 hours in the container. The polymerized laminate was taken out of the container after the completion of the polymerizing step. As a result, a dental mill blank in the form of a desired two-layer laminate was obtained. The obtained dental mill blank had a prism shape of 14.5 mm × 18.0 mm × 14.5 mm, and the amount of the ignition residue was 61 mass%.

Example 6

The inorganic fillers G, H, I, and J (1.1 g each) were spread over a lower punch rod of a rectangular press mold of 14.5 × 18.0 mm in sequence to form a four-layer laminate. The inorganic fillers G, H, I, and J were subjected to tapping for even spreading. Thereafter, an upper punch rod was set at a predetermined position, and uniaxial pressing (pressing pressure: 60 MPa (16 kN), time period: 1 minute) was performed using a press machine. The upper and lower punch rods were removed from the mold, and a molded body having a four-layer structure was taken out. Thereafter, the molded body was placed inside a polyethylene bag, and the polymerizable monomer-containing composition (m) was added to the bag. Then, the pressure in the bag was reduced to impregnate the molded body with the polymerizable monomer-containing composition (m). The bag was allowed to stand still for one day at room temperature under reduced pressure. Then, using a hot air dryer, the bag was heated at 55°C for 18 hours and further heated at 110°C for 3 hours to polymerize the polymerizable monomer. As a result, a dental mill blank in the form of a desired four-layer laminate was obtained. The obtained dental mill blank had a prism shape of 14.5 mm × 18.0 mm × 14.5 mm, and the amount of the ignition residue was 59 mass%.

Example 7

A dental mill blank in the form of a desired four-layer laminate was obtained in the same manner as in Example 6, except that the inorganic fillers K, L, M, and N were used instead of the inorganic fillers G, H, I, and J. The obtained dental mill blank had a prism shape of 14.5 mm × 18.0 mm × 14.5 mm, and the amount of the ignition residue was 59 mass%.

Comparative Examples 1 and 2

Dental mill blanks of Comparative Examples 1 and 2 having a single-layer structure were obtained in the same manner as in Example 1, except that, instead of the inorganic filler A, the inorganic fillers O and P were used, respectively. The obtained dental mill blanks both had a prism shape of 14.5 mm × 18.0 mm × 14.5 mm, and the amount of the ignition residue in each of the dental mill blanks was 60 mass%.

Comparative Example 3

A dental mill blank of Comparative Example 3 having a single-layer structure was obtained in the same manner as in Example 4, except that the inorganic filler Q was used instead of the inorganic filler D and that a paste-like material was prepared by adding 7.2 g of the inorganic filler Q little by little to 2.8 g of the polymerizable monomer-containing composition (m) while kneading. The obtained dental mill blank had a prism shape of 14.5 mm × 18.0 mm × 14.5 mm, and the amount of the ignition residue was 71 mass%.

Comparative Example 4

A dental mill blank of Comparative Example 4 having a desired three-layer layered structure was obtained in the same manner as in Example 6, except that, instead of the inorganic fillers G, H, I, and J, the inorganic fillers R, S, and T were used to provide a laminate including three layers and that the amount of each inorganic filler was set to 2.0 g. The obtained dental mill blank had a prism shape of 14.5 mm × 18.0 mm × 14.5 mm, and the amount of the ignition residue was 70 mass%.

[Table 1]

|  |  |  |  | Added amount of pigment/g | | | | Content of pigment | |
|---|---|---|---|---|---|---|---|---|---|
|  | Shade | Layer | Inorganic filler | Japanese Pharmacopoeia titanium oxide | Iron oxide black | Iron oxide yellow | Iron oxide red | /g[1] | /parts by mass[2] |
| Ex. 1 | A2 | Single layer | A | 0.01600 | 0.00041 | 0.00430 | 0.00052 | 0.02123 | 0.071 |
| Ex. 2 | A2 | Single layer | B | 0.01650 | 0.00045 | 0.00430 | 0.00057 | 0.02182 | 0.073 |
| Ex. 3 | A3 | Single layer | C | 0.01700 | 0.00035 | 0.00650 | 0.00040 | 0.02425 | 0.081 |
| Ex. 4 | A3.5 | Single layer | D | 0.01500 | 0.00060 | 0.00870 | 0.00053 | 0.02483 | 0.083 |
| Ex. 5 | A2 | Two layers | E | 0.01450 | 0.00016 | 0.00226 | 0.00028 | 0.01720 | 0.057 |
|  |  |  | F | 0.01600 | 0.00045 | 0.00430 | 0.00052 | 0.02127 | 0.071 |
| Ex. 6 | A3 | Four layers | G | 0.01550 | 0.00017 | 0.00318 | 0.00023 | 0.01908 | 0.064 |
|  |  |  | H | G and J were mixed at a mixing ratio of 2 : 1 | | | | - | - |
|  |  |  | I | G and J were mixed at a mixing ratio of 1 : 2 | | | | - | - |
|  |  |  | J | 0.01700 | 0.00035 | 0.00650 | 0.00040 | 0.02425 | 0.081 |
| Ex. 7 | A3.5 | Four layers | K | 0.01450 | 0.00030 | 0.00415 | 0.00026 | 0.01921 | 0.064 |
|  |  |  | L | K and N were mixed at a mixing ratio of 2 : 1 | | | | - | - |
|  |  |  | M | K and N were mixed at a mixing ratio of 1 : 2 | | | | - | - |
|  |  |  | N | 0.01500 | 0.00060 | 0.00870 | 0.00053 | 0.02483 | 0.083 |
| Com. Ex. 1 | A2 | Single layer | O | 0.01120 | 0.00032 | 0.00400 | 0.00024 | 0.01576 | 0.053 |
| Com. Ex. 2 | A3 | Single layer | P | 0.01190 | 0.00025 | 0.00554 | 0.00019 | 0.01788 | 0.060 |
| Com. Ex. 3 | A3.5 | Single layer | Q | 0.00011 | 0.00039 | 0.00240 | 0.00029 | 0.00319 | 0.011 |

(continued)

| | Shade | Layer | Inorganic filler | Added amount of pigment/g | | | | Content of pigment | |
|---|---|---|---|---|---|---|---|---|---|
| | | | | Japanese Pharm acopoeia titanium oxide | Iron oxide black | Iron oxide yellow | Iron oxide red | /g[1] | /parts by mass[2] |
| Com. Ex. 4 | A3.5 | Three layers | R | 0.00725 | 0.00030 | 0.00309 | 0.00013 | 0.01077 | 0.036 |
| | | | S | R and T were mixed at a mixing ratio of 1 : 1 | | | | - | - |
| | | | T | 0.01150 | 0.00060 | 0.00824 | 0.00027 | 0.02061 | 0.069 |

1) indicates the amount of the pigment added to the inorganic filler (30 g) 2) The content (parts by mass) of the pigment relative to 100 parts by mass of the inorganic filler was calculated by converting the total amount of the inorganic filler to 100 parts by mass.

[Table 2]

| | Shade | Layer | Inorganic filler | | Lightness L*white | Chromaticity | | Lightness L*black | Chromaticity | | Lightness ΔL* | Chromaticity | | Opalescence value | Opalescent quality | Color tone reproducibility | Aesthetics |
| | | | Type | Content (mass%) | | a*white | b*white | | a*black | b*black | | Δa* | Δb* | | | | |
|---|---|---|---|---|---|---|---|---|---|---|---|---|---|---|---|---|---|
| Ex. 1 | A2 | Single layer | A | 60 | 84.3 | 6.5 | 29.8 | 69.2 | -0.5 | 12.0 | 15.1 | 7.0 | 17.8 | 19.1 | Good | Acceptable | Good |
| Ex. 2 | A2 | Single layer | B | 60 | 83.3 | 6.7 | 30.0 | 68.4 | 0.7 | 12.0 | 14.9 | 6.0 | 18.0 | 19.0 | Good | Good | Good |
| Ex. 3 | A3 | Single layer | C | 60 | 81.6 | 8.9 | 36.5 | 67.0 | 1.4 | 16.7 | 14.6 | 7.5 | 19.8 | 21.1 | Good | Good | Good |
| Ex. 4 | A3.5 | Single layer | D | 61 | 79.6 | 9.9 | 39.4 | 65.6 | 1.8 | 19.4 | 14.0 | 8.0 | 19.9 | 21.5 | Good | Good | Good |
| Ex. 5 | A2 | Two layers | E | 61 | 88.2 | 2.8 | 23.1 | 69.2 | -1.5 | 6.0 | 19.0 | 4.3 | 17.1 | 17.6 | Good | Excellent | Excellent |
| | | | F | 61 | 83.3 | 6.7 | 30.0 | 68.4 | 1.0 | 13.0 | 14.9 | 5.7 | 17.0 | 17.9 | | | |
| Ex. 6 | A3 | Four layers | G | 59 | 87.3 | 2.8 | 29.4 | 69.2 | -1.3 | 9.4 | 18.2 | 4.1 | 20.0 | 20.4 | Good | Excellent | Excellent |
| | | | H | 59 | 85.2 | 5.2 | 32.8 | 68.3 | -0.3 | 12.3 | 17.0 | 5.5 | 20.5 | 21.2 | | | |
| | | | I | 59 | 83.0 | 7.4 | 35.3 | 67.5 | 0.7 | 15.2 | 15.5 | 6.6 | 20.0 | 21.1 | | | |
| | | | J | 59 | 81.6 | 8.9 | 36.5 | 67.0 | 1.4 | 16.7 | 14.6 | 7.5 | 19.8 | 21.1 | | | |
| Ex. 7 | A3.5 | Four layers | K | 59 | 85.5 | 3.8 | 32.4 | 67.9 | -1.0 | 11.7 | 17.6 | 4.8 | 20.7 | 21.3 | Good | Excellent | Excellent |
| | | | L | 59 | 83.1 | 6.5 | 36.4 | 66.9 | 0.1 | 15.6 | 16.2 | 6.3 | 20.8 | 21.7 | | | |
| | | | M | 59 | 80.9 | 8.6 | 38.5 | 66.0 | 1.2 | 18.2 | 14.9 | 7.4 | 20.3 | 21.6 | | | |
| | | | N | 59 | 79.6 | 9.9 | 39.4 | 65.6 | 1.6 | 18.8 | 14.0 | 8.3 | 20.6 | 22.1 | | | |
| Com. Ex. 1 | A2 | Single layer | O | 60 | 86.5 | 3.1 | 26.4 | 70.1 | -0.7 | 8.2 | 16.4 | 3.7 | 18.1 | 18.5 | Good | Poor | Poor |
| Com. Ex. 2 | A3 | Single layer | P | 60 | 83.0 | 4.3 | 31.1 | 67.3 | -0.6 | 11.9 | 15.7 | 4.8 | 19.2 | 19.8 | Good | Poor | Poor |
| Com. Ex. 3 | A3.5 | Single layer | Q | 71 | 81.0 | 5.7 | 32.6 | 66.3 | 0.7 | 16.1 | 14.7 | 5.0 | 16.5 | 17.2 | Poor | Good | Poor |

| | Shade | Layer | Inorganic filler | | Lightness L*<sub>white</sub> | Chromaticity | | Lightness L*<sub>black</sub> | Chromaticity | | Lightness ΔL* | Chromaticity | | Opalescence value | Opalescent quality | Color tone reproducibility | Aesthetics |
|---|---|---|---|---|---|---|---|---|---|---|---|---|---|---|---|---|---|
| | | | Type | Content (mass%) | $L^*_{white}$ | $a^*_{white}$ | $b^*_{white}$ | $L^*_{black}$ | $a^*_{black}$ | $b^*_{black}$ | $\Delta L^*$ | $\Delta a^*$ | $\Delta b^*$ | | | | |
| Com. Ex. 4 | A3.5 | Three layers | R | 70 | 86.3 | 1.9 | 29.4 | 67.4 | -1.0 | 14.3 | 18.9 | 2.8 | 16.4 | 15.4 | Poor | Good | Poor |
| | | | S | 70 | 84.6 | 3.2 | 33.8 | 66.2 | -0.6 | 18.3 | 18.4 | 3.8 | 17.6 | 15.9 | | | |
| | | | T | 70 | 78.8 | 6.6 | 37.3 | 65.0 | 1.5 | 22.4 | 13.8 | 5.5 | 16.6 | 15.7 | | | |

EP 3 903 760 A1

Comparison of the dental mill blanks of Examples 1 to 7 with the dental mill blanks of Comparative Examples 1 to 4 revealed that the dental mill blanks whose chroma levels on the black background ($a^*_{black}$, $b^*_{black}$) were high ($a^* \geq -0.5$, $b^* \geq 12$) exhibited superior color tone reproducibility in the oral cavity and that the dental mill blanks having high opalescence values (17.5 or more) were superior in terms of opalescent quality. These results demonstrate that the dental mill blanks of the present invention, which satisfy both of these conditions, were superior in terms of aesthetics.

INDUSTRIAL APPLICABILITY

A dental mill blank of the present invention can be fabricated, through a milling process, into a highly aesthetic dental prosthesis having an opalescent quality characteristic of natural teeth and also having a sufficient chroma level. According to the present invention, the mill blank can be fabricated into a dental prosthesis that achieves a high level of aesthetics in a dark oral environment even when it has a single-layer structure, and the mill blank can be fabricated into a dental restoration with a higher degree of reproducibility of the aesthetic appearance of a natural tooth when it has a layered structure.

**Claims**

1. A dental mill blank comprising:

    40 mass% or more of an inorganic filler,
    wherein the dental mill blank satisfies the following conditions (1) and (2):

    (1) a specimen with a thickness of 1.20 $\pm$ 0.01 mm has a chromaticity satisfying $-0.5 \leq a^*_{black} \leq 2.5$ and $12.0 \leq b^*_{black} \leq 25.0$ in the L*a*b* color system when measured against a black background; and
    (2) the specimen with a thickness of 1.20 $\pm$ 0.01 mm has an opalescence value of 17.5 or more.

2. The dental mill blank according to claim 1, wherein the dental mill blank has a layered structure comprising two or more layers, and at least one of the layers satisfies the following conditions (1) and (2):

    (1) a specimen with a thickness of 1.20 $\pm$ 0.01 mm has a chromaticity satisfying $-0.5 \leq a^*_{black} \leq 2.5$ and $12.0 \leq b^*_{black} \leq 25.0$ in the L*a*b* color system when measured against a black background; and
    (2) the specimen with a thickness of 1.20 $\pm$ 0.01 mm has an opalescence value of 17.5 or more.

3. The dental mill blank according to claim 1 or 2, wherein the dental mill blank has a layered structure comprising two or more layers, at least one of the layers satisfies the following conditions (1) and (2), and at least one other layer satisfies the following condition (3):

    (1) a specimen with a thickness of 1.20 $\pm$ 0.01 mm has a chromaticity satisfying $-0.5 \leq a^*_{black} \leq 2.5$ and $12.0 \leq b^*_{black} \leq 25.0$ in the L*a*b* color system when measured against a black background;
    (2) the specimen with a thickness of 1.20 $\pm$ 0.01 mm has an opalescence value of 17.5 or more; and
    (3) a specimen with a thickness of 1.20 $\pm$ 0.01 mm has a chromaticity satisfying $-2.0 \leq a^*_{black} \leq 0.0$ and $3.0 \leq b^*_{black} \leq 16.0$ in the L*a*b* color system when measured against a black background.

4. The dental mill blank according to any one of claims 1 to 3, wherein the inorganic filler comprises an inorganic filler having an average particle size of 30 to 700 nm.

5. The dental mill blank according to any one of claims 1 to 4, further comprising an inorganic pigment, wherein the content of the inorganic pigment is 0.070 to 0.100 parts by mass relative to 100 parts by mass of the inorganic filler.

6. The dental mill blank according to claim 5, wherein the inorganic pigment comprises three or more types of inorganic pigments.

7. The dental mill blank according to any one of claims 1 to 6, wherein the inorganic filler comprises an inorganic filler having an average particle size of 30 to 180 nm.

## INTERNATIONAL SEARCH REPORT

| International application No. |
|---|
| PCT/JP2019/051551 |

A. CLASSIFICATION OF SUBJECT MATTER
Int.Cl. A61K6/831(2020.01)i, A61K6/887(2020.01)i
FI: A61K6/027, A61K6/083500

According to International Patent Classification (IPC) or to both national classification and IPC

B. FIELDS SEARCHED

Minimum documentation searched (classification system followed by classification symbols)
Int.Cl. A61K6/831, A61K6/887

Documentation searched other than minimum documentation to the extent that such documents are included in the fields searched
Published examined utility model applications of Japan    1922–1996
Published unexamined utility model applications of Japan   1971–2020
Registered utility model specifications of Japan          1996–2020
Published registered utility model applications of Japan   1994–2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | WO 2018/074605 A1 (KURARAY NORITAKE DENTAL INC.) 26.04.2018 (2018-04-26), claims, examples | 1-7 |
| A | WO 2018/230657 A1 (KURARAY NORITAKE DENTAL INC.) 20.12.2018 (2018-12-20), claims, examples | 1-7 |
| A | JP 11-100305 A (KURARAY CO., LTD.) 13.04.1999 (1999-04-13), claims, examples | 1-7 |
| A | JP 7-196429 A (KURARAY CO., LTD.) 01.08.1995 (1995-08-01), claims, examples | 1-7 |
| A | WO 2018/074583 A1 (YAMAKIN CO., LTD.) 26.04.2018 (2018-04-26), claims, examples | 1-7 |

☒ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28.01.2020 | 10.02.2020 |

| Name and mailing address of the ISA/ Japan Patent Office 3-4-3, Kasumigaseki, Chiyoda-ku, Tokyo 100-8915, Japan | Authorized officer  Telephone No. |
|---|---|

Form PCT/ISA/210 (second sheet) (January 2015)

**INTERNATIONAL SEARCH REPORT**

| International application No. |
| --- |
| PCT/JP2019/051551 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2014/021343 A1 (KURARAY NORITAKE DENTAL INC.) 06.02.2014 (2014-02-06), claims, examples | 1-7 |
| P, A | WO 2019/131787 A1 (KURARAY NORITAKE DENTAL INC.) 04.07.2019 (2019-07-04), claims, examples | 1-7 |
| P, A | JP 2019-98073 A (KURARAY NORITAKE DENTAL INC.) 24.06.2019 (2019-06-24), claims, examples | 1-7 |

Form PCT/ISA/210 (second sheet) (January 2015)

| | | | | |
|---|---|---|---|---|
| **INTERNATIONAL SEARCH REPORT** | | | International application No. | |
| Information on patent family members | | | PCT/JP2019/051551 | |

```
WO 2018/074605 A1  26.04.2018   (Family: none)

WO 2018/230657 A1  20.12.2018   (Family: none)

JP 11-100305 A     13.04.1999   (Family: none)

JP 7-196429 A      01.08.1995   (Family: none)

WO 2018/074583 A1  26.04.2018   (Family: none)

WO 2014/021343 A1  06.02.2014   US 2015/0182315 A1
                                claims, examples
                                EP 2881077 A1
                                CN 104507413 A

WO 2019/131787 A1  04.07.2019   (Family: none)

JP 2019-98073 A    24.06.2019   (Family: none)
```

Form PCT/ISA/210 (patent family annex) (January 2015)

**EP 3 903 760 A1**

## REFERENCES CITED IN THE DESCRIPTION

**Patent documents cited in the description**

- JP 2014161440 A
- WO 2018074605 A1
- JP 2017105764 A
- JP 9003109 A
- JP 10245525 A